# FASCICULE DE BREVET EUROPEEN

(11) **EP 2 046 281 B1**
(45) Date de publication et mention de la délivrance du brevet: **13.08.2014**
(21) Numéro de dépôt: 07788894.9
(22) Date de dépôt: 15.06.2007
(51) Int. Cl.: A61K 8/97, A61Q 19/08

(54) **UTILISATION D'UN EXTRAIT DE GRAINES DE MIMOSA (D'ACACIA DEALBTA, D'ACACIA FARNESIANA OU D'ACACIA DECURRENS) DANS UNE COMPOSITION COSMETIQUE**
VERWENDUNG EINES MIMOSENSAMEN-EXTRAKTS (ACACIA DEALBTA, ACACIA FARNESIANA OU ACACIA DECURRENS) IN EINER KOSMETISCHEN ZUSAMMENSETZUNG
USE OF A MIMOSA SEED EXTRACT (ACACIA DEALBTA, ACACIA FARNESIANA OU ACACIA DECURRENS) IN A COSMETIC COMPOSITION

(30) Priorité: 16.06.2006 FR 0605397; 16.06.2006 FR 0605398
(43) Date de publication de la demande: 15.04.2009
(73) Titulaire: SOCIETE DE RECHERCHE COSMETIQUE, 2557 Luxembourg (LU)
(72) Inventeur: Leclere, Jacques, F-45500 Saint-Gondon (FR)
(74) Mandataire: L'Helgoualch, Jean
(86) Numéro de dépôt international: PCT/FR2007/001000
(87) Numéro de publication internationale: WO 2007/144518

(56) Documents cités:
- EP-A- 1 454 620
- EP-A1- 0 349 469
- WO-A-01/07008
- WO-A-03/075862
- FR-A- 2 695 318
- FR-A- 2 714 605
- JP-A- 2004 352 639
- US-A1- 2005 244 351
- US-A1- 2006 045 894

## Description

La présente invention concerne une nouvelle utilisation dans le domaine cosmétique et/ou dermatologique, et plus particulièrement l'utilisation d'extrait de graines de mimosa pour la préparation d'une composition utilisable en cosmétique et en dermatologie pour les soins et la protection de la peau contre les signes du vieillissement.

La peau comprend des couches superficielles, à savoir l'épiderme, et des couches plus profondes, le derme et l'hypoderme, et chacune possède des propriétés spécifiques permettant à l'ensemble de réagir et s'adapter aux conditions de son environnement. Le derme sert de support à l'épiderme et est principalement constitué de fibroblastes et d'une matrice extracellulaire essentiellement à base de collagène et d'élastine.

Les fibres de collagène contribuent à la tonicité et à l'élasticité de la peau. Avec l'âge, leur renouvellement diminue, ce qui se traduit par un amincissement de la peau, et leur perte progressive d'élasticité entraîne un durcissement de la peau. L'épiderme, qui est constitué de trois types de cellules dont les plus importantes sont les kératinocytes, constitue la couche externe, et joue un rôle fondamental pour assurer la protection et le maintien d'une bonne trophicité. C'est pourquoi de nombreuses compositions ont été mises au point afin de le protéger et d'améliorer les fonctions de la peau.

On sait que le vieillissement de la peau s'accompagne d'un certain nombre de signes et en particulier de la formation de rides plus ou moins profondes et étendues, outre une perte d'élasticité et un amincissement. L'apparition des premières rides, qui peut survenir chez des individus dès l'âge de 30 à 40 ans, est un phénomène qui peut être aggravé par des agressions physiques ou chimiques provenant de la pollution, de l'exposition aux rayonnements ultraviolets ou des modes de vie qui accélèrent le vieillissement cutané.

A l'échelle de la peau, le vieillissement provoque notamment une diminution des synthèses protéiques (collagène, élastine), ainsi qu'une élévation des métalloprotéinases de type MMP-3.

D'une manière générale, le traitement et la prévention des signes du vieillissement cutané sont l'objet de nombreux travaux et études depuis des années, notamment pour mettre au point des compositions susceptibles de favoriser la restructuration tissulaire, et en particulier la néosynthèse d'éléments constitutifs de la peau comme le collagène. Par "néosynthèse", on entend la synthèse du collagène qui se forme uniquement en cas de besoin, par exemple pour le comblement des rides.

Par ailleurs, on sait que, pour être acceptées par les utilisateurs, les compositions cosmétiques et/ou dermatologiques destinées au traitement et à la prévention des affections de la peau par application topique doivent être agréables à utiliser et doivent présenter de bonnes propriétés physiques, notamment de consistance et d'onctuosité, tout en garantissant une efficacité satisfaisante et en évitant les inconvénients tels que tiraillements, irritations ou démangeaisons, que peuvent occasionner certains principes actifs.

Dans l'état de la technique, par exemple le brevet FR-A-2.761.607 décrit une composition dermatologique destinée au traitement des symptômes du vieillissement de la peau, comportant un dérivé de silanol méthylé et un dérivé d'une protéine végétale hydrolysée, additionnée le cas échéant d'un dérivé de vitamine C. Le brevet EP-A-662.319 décrit des compositions cosmétiques et dermatologiques comprenant un céramide comme agent apaisant pour compenser l'effet irritant du principe actif anti-vieillissement. Le brevet FR-A-2.783.169 décrit l'utilisation de pentapeptides du type LysThr-Thr-Lys-Ser dans des compositions topiques pour favoriser la synthèse du collagène et des glycosaminoglycannes, et par conséquent la régénération cutanée.

On connaît également, du brevet FR-A-2 848 116, une composition cosmétique et/ou dermatologique à base d'inhibiteurs de métallo-protéinases matricielles, en particulier d'extrait de Siegesbeckia, et de lipopeptides permettant le traitement et la prévention des signes du vieillissement cutané tels que l'apparition de rides et la perte d'élasticité de la peau.

Enfin la demande internationale WO 0107008 décrit une composition topique destinée à augmenter le taux de collagène dans les cellules de la peau comprenant des dérivés phénoliques extraits de *Mimosa pudica,* ledit extrait étant obtenu à partir des parties aériennes de la plante, sans limitation, y compris les graines.

Cependant, il existe toujours un besoin accru de trouver des compositions topiques alternatives permettant de lutter efficacement contre le vieillissement cutané, et notamment de trouver des compositions topiques à base de végétaux appropriés, particulièrement provenant de plantes aisément accessibles et connues pour leurs facultés de résistance.

Suivant la présente invention, on a trouvé qu'il était possible d'agir efficacement contre les signes du vieillissement cutané en utilisant des compositions topiques à base d'extraits de graines de mimosa.

La présente invention a donc pour objet une nouvelle utilisation d'un extrait de graines de mimosa, pour la préparation d'une composition topique à usage cosmétique et/ou dermatologique, permettant de lutter contre les signes du vieillissement cutané tels que l'apparition de rides et la perte d'élasticité de la peau.

La présente invention a encore pour objet un procédé cosmétique pour combattre les signes du vieillissement cutané, consistant à appliquer sur les zones de la peau affectées une composition contenant une quantité efficace d'extrait de graines de mimosa.

Le mimosa de l'invention appartient aux espèces Acacia dealbata, Acacia farnesiana et Acacia decurrens, et présente l'avantage d'être extrêmement résistant. Il est en effet capable de repousser aisément, qu'il ait subi le gel ou au contraire une très forte chaleur. En outre, ses graines sont très riches en protéines et en globulines, et possèdent par conséquent un pouvoir tenseur et nutritif. Parmi ces trois espèces, l'Acacia dealbata est préféré, dans la mesure où ses graines sont faciles à se procurer.

Dans certaines médecines traditionnelles, les fleurs de l'espèce Acacia farnesiana sont utilisées pour traiter les maux de tête et les indigestions, tandis que des décoctions de cosses sont employées pour traiter la dysenterie et les inflammations de la peau.

L'invention a également pour objet une utilisation combinée d'extrait de graines de mimosa et d'extrait de souci pour la préparation d'une composition utilisable en cosmétique et en dermatologie, pour les soins et la protection de la peau contre les signes du vieillissement, ainsi qu'une composition cosmétique utile contre les signes du vieillissement cutané, comprenant en combinaison un extrait de graines de mimosa et un extrait de souci.

Le souci (Calendula officinalis, ou "souci des jardins" et Calendula arvensis, ou "souci des champs") est déjà connu pour posséder des propriétés thérapeutiques.

En particulier, il a été montré que l'espèce Calendula officinalis possède, en usage interne, des propriétés sudorifiques, emménagogues, antispasmodiques et cholérétiques, et, en usage externe, des propriétés vulnéraires et anti-inflammatoires. Des propriétés ocytociques, hypotensives et vasodilatatrices ont également été mises en évidence. En France, le Calendula officinalis est principalement utilisé en homéopathie ou en mélange dans des tisanes. Dans d'autres pays tels que l'Allemagne, les Pays-Bas et la Russie, il est utilisé dans des mélanges théiformes ou, en usage externe, contre les contusions, les ulcères, les gelures et les furoncles.

Il a été montré que le souci pouvait être efficace contre l'acné, et en tant qu'agent adoucissant.

L'espèce Calendula arvensis possède également des propriétés emménagogues, vaso-dilatatrices et hypotensives.

Les études effectuées par la demanderesse ont montré de manière surprenante que l'utilisation d'une quantité efficace d'un extrait de graines de mimosa, provenant en particulier des espèces Acacia dealbata, Acacia farnesiana ou Acacia decurrens, éventuellement combinée avec une quantité efficace d'extrait de souci (Calendula officinalis ou Calendula arvensis), avait notamment pour effet de favoriser fortement la synthèse des collagènes, ce qui entraîne un épaississement de l'épiderme et par conséquent un aplanissement des rides, d'améliorer la néosynthèse des collagènes de type I et de type III et de diminuer le rapport collagène I/collagène III, et également de diminuer l'expression des métalloprotéinases III (MMP-3) aboutissant à un meilleur stockage de collagène néoformé. Les résultats des tests in vitro mettant en évidence ces propriétés sont détaillés ci-après.

Les extraits de mimosa utilisés dans les compositions suivant l'invention sont obtenus à partir de graines de mimosa, par exemple par macération dans l'eau. A cet effet, les graines pulvérisées (10 à 20 g pour la préparation de 100 g d'extrait) sont mises à macérer dans de l'eau déminéralisée à une température comprise entre 40 et 50°C pendant 48 heures, en agitant de temps en temps. Le produit est ensuite filtré, le gâteau est exprimé, et les liqueurs sont réunies et clarifiées. La quantité est ajustée avec de l'eau, et on ajoute 50% d'eau ainsi que 0,5% de conservateur, le tout est complété à 100 et filtré par filtration stérilisante (pourcentages exprimés en poids).

Typiquement, on peut utiliser un extrait aqueux de graines de mimosa présentant la composition suivante :
- matières sèches 2 à 4%
- densité à 22°C 0,980 à 1,020
- indice de réfraction à 22°C 1,330 à 1,350
- pH 5,7 à 7,2
- teneur en protéines totale >0,1%
(par rapport aux matières sèches)

Les compositions topiques incluent typiquement entre 0,001 et 15% en poids d'extrait de mimosa par rapport au poids total de la composition. De façon avantageuse, une telle composition comprend 2% en poids d'extrait de mimosa par rapport au poids total de la composition.

Comme indiqué ci-dessus, il est important d'utiliser comme source d'extrait les graines de mimosa à l'exclusion de toute autre partie de la plante, en raison de la composition particulière des graines, qui permet de procurer les propriétés spécifiques des compositions de la présente invention.

Lorsque l'extrait de graines de mimosa est utilisé en combinaison avec des extraits de souci, les extraits de souci sont avantageusement obtenus à partir de pétales de fleurs de souci, par exemple par macération hydro-glycolique des fleurs pulvérisées. La macération peut être réalisée dans un mélange butylène-glycol/eau. A cet effet, un mode de réalisation consiste à faire macérer des pétales de souci pulvérisés dans de l'eau à une température comprise entre environ 45°C et 50°C pendant 48 heures, puis à filtrer et à exprimer le gâteau. Les liqueurs sont ensuite réunies et l'on ajoute 50% de butylène glycol. La mise en oeuvre est généralement de 10g / 100g de fleurs sèches.

Typiquement, on peut utiliser un extrait de souci présentant la composition suivante :
- eau / butylène glycol 50/50
- matières sèches 1,5 à 3,0%
- flavonoïdes 0,2 à 3,0%
(sur matière sèche, exprimé en rutine)

Les matières sèches comprennent notamment des caroténoïdes, des flavonoïdes, des saponosides triterpéniques dérivés de l'acide oléanolique, des alcools triterpéniques et de la calendine.

Parmi les caroténoïdes, on peut citer le carotène, le lycopène, la violaxanthine, la flavoxanthine. Les flavonoïdes comprennent des hétérosides de l'isorhamnitol (tétrahydroxy-3,5,7,4' méthoxy 3' flavone). Parmi les saponosides triterpéniques dérivés de l'acide oléanolique, on peut citer l'acide glycuronique, le glucose, le galactose. Des exemples d'alcools triterpéniques sont l'arnidiol, le faradiol, le taraxastérol, l'a et β-amyrine.

Les compositions topiques selon l'invention, lorsqu'elles comprennent de l'extrait de souci, incluent typiquement entre 0,001 et 0,5% en poids d'extrait de souci par rapport au poids total de la composition.

Le pH de la composition selon l'invention est de préférence compris entre 6 et 6,5, et peut être ajusté, selon les compositions, par addition d'un acide tel que l'acide citrique ou d'une base telle que l'hydroxyde de sodium.

Il peut être avantageux, suivant une variante de la présente invention, d'incorporer dans la composition des principes actifs ou ingrédients auxiliaires choisis pour leurs propriétés complémentaires, afin de renforcer les effets anti-âge de la composition. Ainsi il est particulièrement avantageux de les combiner avec des quantités appropriées d'une ou plusieurs substances choisies parmi la Vitamine A palmitate, la Vitamine C ou ses dérivés, les protéines de graines d'amarante (Amarantus caudatus), les globulines de pois ou les mucilages tels que le mucilage de fruits de baobab afin d'obtenir un effet tenseur de la peau, l'huile de Calophylum afin de renforcer l'effet anti-rides, l'huile d'Echium pour son effet anti-inflammatoire, un palmitoyl pentapeptide-3 tel que le Matrixyl^{®} ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que de manière générale toute association avec un céramide, une association comprenant du Matrixyl^{®} et un extrait de Siegesbeckia orientalis, les polyphénols, et notamment les polyphénols de cacao, l'acide oléanolique, une antihyaluronidase telle que Echinacine B afin de générer un maximum d'eau liée, l'Imperata cylindrica, par exemple MOIST 24^{®} de la société Sederma, afin de favoriser l'hydratation de la peau par modification de la pression osmotique, un extrait de graines de lotus, de graines de coquelicot, de racine de guimauve, les peptides d'Hibiscus de type Myoxynol^{®} (commercialisé par les Laboratoires Sérobiologiques), les extraits de fenouil, les peptides d'origine végétale de type Argireline^{®} (commercialisés par la société Lipotec).

Il est particulièrement avantageux d'utiliser, dans la composition de l'invention, un extrait de graines de mimosa en combinaison avec un extrait de souci et un palmitoyl pentapeptide-3 tel que le Matrixyl^{®} pour obtenir un effet antirides renforcé.

Les compositions peuvent également contenir diverses substances et excipients choisis en fonction de leurs propriétés connues et de la forme galénique envisagée. Ainsi, on peut incorporer des agents hydratants, des agents calmants, des agents émulsionnants, des tensioactifs, des épaississants, des gélifiants, des agents viscosants, des conservateurs, des antioxydants, des parfums, des huiles, des lipides, un solvant spécifique ainsi que de l'eau et divers additifs destinés à améliorer les propriétés physiques des compositions. On peut encore avantageusement incorporer des filtres ou écrans solaires choisis en fonction du degré de protection recherché.

L'agent hydratant peut être choisi parmi les produits connus dans la préparation de compositions utilisables en cosmétologie et en dermatologie, et par exemple on peut utiliser un polyol, la glycérine (glycérol et des dérivés de glycérol), des alkylène polyols tels que le polyéthylène glycol, le sorbitol, le maltitol, le penta-érythritol, les polyacrylates et polyméthacrylates de glycéryle, les mucopolysaccharides tels que l'acide hyaluronique, des dérivés du chitosan et des dérivés de l'acide pyrrolidone carboxylique.

Les compositions suivant la présente invention peuvent être présentées sous les formes classiquement utilisées pour une application topique, c'est-à-dire sous forme de gel, lotion, sérum, émulsion (en particulier crème ou lait), masque, stick, pommade ou patch, contenant les composants décrits ci-dessus, et des excipients et supports usuels compatibles et pharmaceutiquement acceptables. Les formes préférées sont les sérums, les crèmes, les gels ou les patches, sous forme vectorisée ou non.

Ces formes d'administration par voie topique sont préparées par les techniques connues, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout.

Dans le cas des émulsions, la phase grasse peut représenter entre 10 et 60% environ du poids de la composition, la phase aqueuse entre 10 et 80% environ et l'agent émulsionnant entre 2 et 20%, le reste étant constitué par les composants de base de la composition selon l'invention et par les autres substances mentionnées ci-dessus. Si un agent hydratant est incorporé, il est avantageusement introduit dans la phase aqueuse lors de la préparation de l'émulsion. La teneur en agent hydratant est généralement comprise entre 0,1 et 10% en poids par rapport au poids total de la composition.

Les exemples qui suivent illustrent la présente invention, sans en limiter la portée.

Des exemples de formulations sont rapportés aux exemples 1 à 3. Dans ces exemples, les parties sont exprimées en poids, sauf indication contraire.

L'exemple 4 est relatif à des tests d'activité sur une co-culture d'épidermes reconstitués/fibroblastes humains, qui ont permis d'évaluer l'effet régénérant d'un extrait aqueux de mimosa (Acacia dealbata), dénommé ci-après par le produit "extrait de mimosa".

### Exemple 1

| Crème anti-rides | |
|---|---|
| Sodium heptonate | 0,1 |
| Pentylène glycol | 5,0 |
| Capryl glycol | 3,0 |
| Octyl glycérol | 0,3 |
| Caprylyl glycocolle | 1,5 |
| Extrait de graines de Mimosa | 4,0 |
| Extrait de fleurs de Souci | 0,5 |
| Extrait de racine de Guimauve | 5,0 |
| Stéaryl glucoside | 3,0 |
| PEG-20 stéaryl glucoside | 2,5 |
| Alcool béhénylique | 2,5 |
| Huile d'Inca-Inchi | 3,0 |
| Beurre de mangue | 1,5 |
| Huile de noyaux d'abricot | 1,0 |
| Acide oléanolique | 0,5 |
| Palmitate de Vitamine A à 1 million U.I./g | 0,5 |
| Palmitate de Vitamine C | 0,15 |
| Tocophérol naturel | 0,7 |
| Eau déminéralisée | 10,0 |
| Hyaluronate de sodium | 0,1 |
| Huiles essentielles naturelles | 0,3 |
| Acide citrique et hydroxyde de sodium : quantité suffisante pour atteindre pH = 6 | |
| Gomme xanthane | 0,5 |
| Eau déminéralisée QSP | 100,0 |

### Exemple 2

| Crème anti-rides fluide | |
|---|---|
| Matrixyl 3000^{®} | 3,0 |
| Extrait de fleurs de souci | 0,5 |
| Extrait de graines d'Acacia dealbata | 2,0 |
| Mucilage de fruits de Baobab | 5,0 |
| Extrait de graines de Lotus Bleu | 1,0 |
| Extrait de racine de guimauve | 2,5 |
| Escine | 0,1 |
| Pentylène glycol | 5,0 |
| Capryl glycol | 3,0 |
| Octyl glycérol | 0,3 |
| Sodium heptonate | 0,1 |
| Acide 10-hydroxydécanoïque | 0,15 |
| PEG-20 sorbitan monostéarate | 3,5 |
| Sorbitan monostéarate | 2,5 |
| Huile d'Echium | 3,0 |
| Perhydrosqualène | 3,0 |
| Huile de camélia | 1,0 |
| Palmitate de Vitamine C | 0,15 |
| Tocophérol | 0,7 |
| Monostéarate de glycérol | 2,0 |
| Alcool cétostéarylique | 0,3 |
| Eau déminéralisée | 10,0 |
| Protéines d'Amande hydrolysées | 5,0 |
| Globulines de pois | 3,0 |
| Extrait de miel | 3,0 |
| Essence d'Ylang-Ylang | 0,1 |
| Essence de Géranium | 0,1 |
| Essence de Cyprès | 0,025 |
| Essence d'Oranges Amères | 0,05 |
| Essence de romarin | 0,025 |
| Eau déminéralisée QSP | 100,0 |

### Exemple 3

| Crème raffermissante pour l'ovale du visage | |
|---|---|
| Pentylène glycol | 5,0 |
| Capryl glycol | 3,0 |
| Octyl glycérol | 0,3 |
| Sodium heptonate | 0,1 |
| Lécithine de Soja hydrogénée | 3,0 |
| Acide palmitique | 0,5 |
| Alcools comprenant entre 12 et 16 atomes de carbone | 0,5 |
| Glycérine | 2,0 |
| Extrait de graines de Mimosa | 10,0 |
| Extrait de Barbatimao | 1,5 |
| Sulfate de dextran | 0,5 |
| Escine | 0,1 |
| Caféine | 1,0 |
| Hyaluronate de sodium | 0,1 |
| Extrait de Fomes officinalis | 0,5 |
| Huile de Calophyllum Inophyllum | 0,5 |
| Huile d'Amandes d'Abricot | 2,0 |
| Perhydrosqualène | 1,5 |
| Beurre de Karité | 0,5 |
| Alcool béhénylique | 0,7 |
| Alcool butylique | 0,5 |
| Mucilage de figues | 3,0 |
| Tocophérol | 0,5 |
| Essence de Géranium | 0,1 |
| Eau déminéralisée QSP | 100,0 |

### Exemple 4

Le but de l'étude décrite ci-dessous est d'évaluer le potentiel régénérant d'un extrait aqueux de graines de mimosa (Acacia dealbata) sur une co-culture d'épidermes reconstitués/fibroblastes humains. Pour cela, les dosages suivants sont réalisés :
- dosage des collagènes totaux
- dosage du rapport collagène type I / collagène type III
- dosage des métalloprotéinases 3 (MMP-3).

### 4-1. PROTOCOLE EXPERIMENTAL

### 4.1.1. Epidermes reconstitués, modèle SKINETHIC™

Des kératinocytes d'origine humaine sont ensemencés sur des filtres en polycarbonate de 0,5 cm² dans un milieu défini (MCDB 153 modifié) et supplémenté. Les cellules sont cultivées pendant 14 jours à l'interface air/liquide, le milieu de culture étant changé tous les deux jours. Les épidermes ainsi formés sont utilisés pour la réalisation de l'étude à partir du 17^{e} jour de la culture.

Pour évaluer la viabilité cellulaire, un essai préliminaire est effectué pour chaque produit étudié afin de déterminer les temps de contact composition/épiderme n'entraînant pas de cytotoxicité.

L'essai est conduit en duplicate à chaque temps expérimental : 6 et 24 heures.
- Lot 1 : deux épidermes témoins ne recevant pas de produit
- Lot 2 : deux épidermes traités recevant un extrait de mimosa à 0,2% (2 µl/cm²)
- Lot 3 : deux épidermes traités recevant un extrait de mimosa à 0,5% (2 µl/cm²)
- Lot 4 : deux épidermes traités recevant un extrait de mimosa à 1% (2 µl/cm²).

La viabilité cellulaire est d'abord étudiée par réaction colorimétrique au sel de tétrazonium (MTT).

Le sel de tétrazolium a la propriété d'être réduit en cristaux bleux de formazan par la succinate déshydrogénase mitochondriale des cellules. CeL enzyme, qui joue un rôle important dans le cycle de Krobs, catalyse la déshydrogénation du succinate en fumarate. C'est l'activité de cet enzyme, flavoprotéine très fortement liée à la membrane interne mitochondriale, qui est mesurée, par réduction du MTT. Par un dosage spectrophotométrique, il est possible de déterminer la toxicité d'une population cellulaire donnée. En effet, l'absorbance est directement reliée à l'activité des succinates déshydrogénases, elle-même liée à la toxicité cellulaire.

0,15 ml de milieu de culture contenant 10% vol/vol de solution de MTT est pipetté sous chacun des filtres/support de culture. Après une incubation de 30 minutes à température ambiante, la couleur des différentes cultures est observée et notée. Les cultures doivent être de couleur bleue/pourpre intense, preuve de la viabilité des cellules de l'épiderme.

La viabilité cellulaire est également étudiée par examen histologique. Pour cela, les épidermes fixés dans une solution contenant 10% de formaldéhyde sont inclus dans des blocs en paraffine. Les coupes verticales de 4 microns sont colorées à l'hématoxyline/éosine et photographiées sous un microscope optique.

### 4.1.2. Fibroblastes humains en culture

On utilise la méthode de la digestion enzymatique permettant d'obtenir des primocultures de fibroblastes à partir d'une biopsie de peau humaine. Les essais sont réalisés sur des fibroblastes entre le 2^{e} et le 4^{e} passage afin d'assurer une reproductibilité entre les différentes expériences.

La cytotoxicité du produit « extrait de mimosa » est évaluée par le test de réduction au bleu de Formazan (MTT) après 24 heures de traitement des cellules avec le produit étudié.

L'essai est réalisé en effectuant, pour chaque lot, 5 mesures de la densité optique à 570nm.

La constitution des lots est la suivante :
- Lot 1 : témoin négatif non traité
- Lot 2 : traité par un extrait de mimosa à 0,2%
- Lot 3 : traité par un extrait de mimosa à 0,5%
- Lot 4 : traité par un extrait de mimosa à 1%.

Après 24 heures d'incubation des cellules avec les différentes concentrations du produit à l'étude, elles sont mises en contact avec du MTT pendant 3 heures à 37°C, puis lysées avec du DMSO. La densité optique est déterminée à 570 nm à l'aide d'un spectrophotomètre après homogénéisation de la coloration.

### 4.1.3. Co-culture Epidermes reconstitués / fibroblastes humains en culture

Les cultures de fibroblastes sont établies à partir de peau de prépuces humains récoltés lors de circoncisions et sont amplifiées en milieu de culture RPMI 1640 supplémenté par du sérum de veau foetal, de la L-glutamine et de la gentamycine. Les essais sont réalisés sur des fibroblastes, entre le 2^{e} et le 4^{e} passage, afin d'assurer une reproductibilité entre les différentes expérimentations. Les fibroblastes sont ensemencés dans des plaques 24 puits à raison de 10⁵ cellules par ml. Ils sont ensuite incubés à 37°C et 5% de CO₂. Après 24 heures d'incubation, les cellules sont mises en contact avec les épidermes reconstitués traités avec l'extrait de mimosa à raison de 2 µl/cm².

### 4.1.3.1. Dosage de la néosynthèse des collagènes

### Dosage quantitatif des collagènes totaux

L'essai est conduit en triplicate après 24 heures de traitement.

La constitution des lots est la suivante :
- Lot 1 : 2 épidermes/fibroblastes témoins ne recevant aucun produit
- Lot 2 : 2 épidermes/fibroblastes traités recevant un extrait de mimosa à 0,2% (2 µl/cm²)
- Lot 3 : 2 épidermes/fibroblastes traités recevant un extrait de mimosa à 0,5% (2 µl/cm²)
- Lot 4 : 2 épidermes/fibroblastes traités recevant un extrait de mimosa à 1% (2 µl/cm²).

Après le temps d'incubation, les fibroblastes sont récupérés par centrifugation. Les culots sont digérés par les collagénases (1 mg/culot cellulaire) dans l'acide acétique 0,5 ml/l pendant 24 heures à 4°C.

Après centrifugation à 10 000 g, les collagènes sont précipités par le chlorure de sodium (NaCl) à 1 M, le précipité est resuspendu puis dialysé.

Les acides aminés primaires sont dérivés par l'acide ophtaldéhyde (OPA) éliminant ainsi leur interférence. L'hydroxyproline et la proline sont alors dérivées par le NBD-Cl par couplage des groupement aminés au NBD-Cl. Le NBD-Hyp est séparé et identifié par HPLC en phase inverse. Pour la mise au point de la séparation des dérivés d'acides aminés, un couplage au NBD-CL d'un standard contenant l'hydroxyproline est d'abord réalisé.
- L'hydroxyproline est dosée par mesure de la fluorescence après séparation par HPLC en phase inverse :
   o Injecteur automatique
   o Colonne Ultrasep C18 (30cm*0,18cm)
   ∘ 6 µm de porosité
   o Détecteur de fluorescence

   La phase mobile est constituée d'un mélange d'acétonitrile/tampon phosphate de sodium 0,1 mol/l, pH 7,2 (9:91 v/v), le débit est réglé à 1 ml/min, l'élution est réalisée en mode statique et le cycle est de 10 minutes. La phase mobile est préalablement filtrée, puis dégazée avant utilisation.
- Les solutions sont préparées de la façon suivante :
   NBD-Cl : 25 mmol dissous dans le méthanol,
   OPA = 150 mmol/l dissous dans du méthanol,
   Tampon phosphate = 0,4 mmol/l, pH ajusté à 7,2.

La gamme étalon est préparée à partir d'une solution d'hydroxyproline à 50 mg/l. Les dilutions successives permettent d'obtenir des solutions allant de 0,5 à 40 mg/l.

La dérivatisation et l'établissement de la courbe d'étalonnage sont effectués à partir de 10 µl d'une solution étalon à différentes concentrations mélangées avec 10 µl du tampon. Après addition de 5 µl d'OPA et agitation, les tubes sont gardés 5 minutes à température ambiante puis 10 µl de la solution NBD-Cl sont ajoutés. Le dérivât se fait à 60°C, au bain marie, pendant 3 minutes, à l'abri de la lumière. Les tubes sont ensuite retirés et la coloration orange permet de vérifier la dérivatisation. Ils sont, par la suite, mis dans la glace afin d'assurer le refroidissement. 50 µl de ce mélange sont ensuite injectés dans la colonne afin d'obtenir la courbe d'étalonnage qui doit être linéaire.
Les échantillons sont traités de la même façon.
Dosage qualitatif des collagènes de type I et de type III.

### Calcul du rapport collagène de type I / collagène de type III

L'essai est conduit en triplicate après 24 heures de traitement.

La constitution des lots est la suivante :
- Lot 1 : 2 épidermes/fibroblastes témoins ne recevant aucun produit ;
- Lot 2 : 2 épidermes/fibroblastes traités recevant un extrait de mimosa à 0,2% (2 µl/cm²) ;
- Lot 3 : 2 épidermes/fibroblastes traités recevant un extrait de mimosa à 0,5% (2 µl/cm²)
- Lot 4 : 2 épidermes/fibroblastes traités recevant un extrait de mimosa à 1% (2 µl/cm²).

Après le temps d'incubation, un marquage isotopique est réalisé en présence de :
- acide ascorbique : 50 µg/mL ;
- glutamine : 2 mM ;
- sérum de veau foetal : 1% v/v ;
- [³H]-Proline (réf. TRK323, Amsterdam) : 50 µCi/puits ;
- l'extrait de mimosa aux différentes concentrations.

Un volume de chaque échantillon contenant le même taux de radioactivité (100 000 dpm) est prélevé, auquel est ajouté 7,5% (v/v) final de β-mercaptoéthanol (Merck), puis dénaturé pendant 3 minutes à 100°C. Les échantillons sont supplémentés par 3 µl de bleu de bromophénol. Les échantillons sont passés sur un gel (SDS PAGE) à 7,5% d'acrylamide.

Après décoloration du gel, les bandes correspondant aux différentes chaînes collagéniques sont découpées et placées dans des tubes à scintillation en présence de 500 µl de Soluène (Packard).

Les bandes sont dissoutes à 60°C pendant 18 heures. La radioactivité est ensuite mesurée en scintillation liquide (compteur β Kontron).

### 4.1.3.2. Etude de l'effet du produit sur la suppression de la MMP-3 (Metalloprotéinase 3)

A la fin du temps d'incubation (24 heures), les milieux de culture sont prélevés, et le dosage de la MMP-3 est réalisé conformément aux protocoles décrits dans les kits de dosage (Kit de détection de la Métalloprotéinase 3 (MMP-3) : Interchim).

### 4.2.RESULTATS

### 4.2.1. Evaluation de la cytotoxicité

### - Epidermes reconstitués

L'évaluation de la cytotoxicité par réaction colorimétrique au sel de tétrazonium (MTT) est réalisée en fonction des différentes concentrations du produit « extrait de mimosa », après 24 heures de contact. Les résultats sont regroupés dans le tableau ci-dessous :

| | Densité optique | % |
|---|---|---|
| Témoin | 1,210±0,03 | - |
| Mimosa (0,2%) | 1,225±0,04 | n.s. |
| Mimosa (0,5%) | 1,202±0,05 | n.s. |
| Mimosa (1%) | 1,214±0,03 | n.s. |

### n.s. : non significatif

Les épidermes reconstitués traités par les différentes concentrations du produit « extrait de mimosa » à raison de 2 µl/cm² montrent une couleur bleue/pourpre intense, preuve de la viabilité des cellules de l'épiderme.

Par examen histologique, il apparaît que le produit « extrait de mimosa » déposé à raison de 2 µl/cm² sur des épidermes reconstitués traités pendant 48 heures, comparativement à des épidermes témoins, n'induit aucune toxicité. Les images histologiques, après coloration HES, d'épidermes traités par les produits sont comparables à celles des épidermes témoins et répondent aux paramètres histologiques normaux.

### - Fibroblastes humains en culture

L'évaluation de la cytotoxicité par réaction colorimétrique au sel de tétrazonium (MTT) est réalisée en fonction des différentes concentrations du produit « extrait de mimosa », après 24 heures de contact. Les résultats sont regroupés dans le tableau ci-dessous :

| | Densité optique | % |
|---|---|---|
| Témoin | 0,330±0,01 | - |
| Mimosa (0,2%) | 0,345±0,02 | n.s. |
| Mimosa (0,5%) | 0,355±0,01 | n.s. |
| Mimosa (1%) | 0,356±0,03 | n.s. |

### n.s. : non significatif

Les résultats obtenus montrent que le produit « extrait de mimosa » n'entraîne aucune cytotoxicité aux différentes concentrations testées.

### 4.2.2. Evaluation de la néosynthèse des collagènes totaux

Les résultats des mesures des collagènes totaux obtenus dans la couche cellulaire et dans le milieu de culture sont regroupés dans le tableau ci-dessous.

| | Collagènes totaux dans la couche cellulaire en [³H] dpm/10⁶ cellules | % | Collagène total dans le milieu de culture en [³H] dpm/10⁶ cellules | % |
|---|---|---|---|---|
| Témoin | 4 852 648 ± 244 025 | - | 6 758 622 ± 86 538 | - |
| Mimosa (0,2%) | 5 140 312 ± 122 443 | +5 ns | 6 912 438 ± 148 315 | ns |
| Mimosa (0,5%) | 6 012 412 ± 85 319* | +24 | 7 819 413 ± 82 456* | +16 |
| Mimosa (1%) | 6 512 538 ± 25 180* | +34 | 8 136 461 ± 68 395* | +20 |

| | | | | |
|---|---|---|---|---|
| *n.s. : non significatif* **Significativement différent par rapport au témoin (p<0,01) (wilcoxon Rank Sum* Test) | | | | |

Les résultats montrent que le produit "extrait de mimosa" aux concentrations 0,5% et 1% entraîne une induction de la néosynthèse des collagènes totaux au niveau de la couche cellulaire, respectivement de 24 et 34%. Cette induction de la néosynthèse des collagènes se traduit par une augmentation du relargage des collagènes dans le milieu de culture (+16 et +20%).

### 4.2.3. Evaluation qualitative de la synthèse du collagène calcul du rapport collagène type I/collagène type III

Les résultats sont regroupés dans le tableau ci-dessous.

| | Collagène type I | Collagène type III | Rapport collagène type I/collagène type III |
|---|---|---|---|
| Témoin | 6302 ± 421 | 2080 ± 148 | 3,03 |
| Mimosa (0,2%) | 5945 ± 312 | 1988 ± 387 | 2,99 |
| Mimosa (0,5%) | 7348 ± 402 | 3126 ± 504 | 2,55 |
| Mimosa (1%) | 7546 ± 982 | 3559 ± 857 | 2,12 |

Les résultats montrent que le rapport collagène type I / collagène type III est voisin de 3 dans la couche cellulaire témoin, ce qui implique un dépôt de collagène I trois fois supérieur au collagène III. Ce rapport est très voisin de celui montré pour le derme humain in vivo. Le produit "extrait de mimosa" aux concentrations 0,5% et 1% induit une augmentation des collagènes I et III avec une diminution du rapport collagène type I/collagène type III, ce qui implique une augmentation du collagène III.

### 4.2.4. Dosage des métalloprotéinases 3 (MMP-3)

Le dosage des MMP3 a été réalisé par des kits ELISA au niveau du milieu de culture.
Les résultats sont reportés dans le tableau ci-dessous :

| | Concentration (MMP3) (pg/ml) | % |
|---|---|---|
| Témoin | 836±33 | - |
| Mimosa (0,2%) | 670±21* | -20 |
| Mimosa (0,5%) | 605±38* | -28 |
| Mimosa (1%) | 492±30* | -41 |

| | | |
|---|---|---|
| **significativement différent par rapport au témoin p<0,01 (wilcoxon Rank Sum Test)* | | |

Les résultats obtenus montrent que le produit "extrait de mimosa" aux concentrations 0,2%, 0,5% et 1%, diminue l'expression de la métalloprotéinase 3 respectivement de 20%, 28% et 41%.

### Conclusion

Ainsi, dans les conditions expérimentales retenues et au vu des résultats obtenus, il apparaît que le produit "extrait de mimosa" présente un effet régénérant de la matrice extracellulaire des fibroblastes humains.

Cet effet se traduit par une augmentation de la néosynthèse des collagènes totaux avec une diminution du rapport collagène type I/collagène type III, ainsi que par une diminution de l'expression des métalloprotéinases 3 (MMP-3). Il est à noter que l'augmentation de synthèse des collagènes est une augmentation du collagène de type III, qui se produit habituellement dans une peau jeune, le collagène de type I n'étant pas diminué. Il s'agit donc bien d'une néosynthèse de collagène jeune dans un rapport favorable.

Ces résultats démontrent l'efficacité de l'extrait de mimosa selon l'invention pour la prévention et le traitement des signes du vieillissement cutané.

### Combinaison "mimosa - souci"

Une étude in vivo a été faite pour mesurer l'effet sur les rides faciales d'une composition suivant l'invention associant un extrait de graines de mimosa, un extrait de souci et du Matrixyl sur les rides comme dans l'Exemple 2.

Les mesures sont faites au 1^{er} jour (à 2 H), puis à 28 jours et à 56 jours, après application quotidienne de la composition sur le visage, matin et soir, par 30 volontaires de sexe féminin. Les mesures sont faites par prise d'empreinte du relief cutané au niveau du coin de l'oeil, par application d'un gel Silflo® qui polymérise in situ et fournit après décollement une empreinte des irrégularités de la peau qui sont analysées sur images numérisées après éclairages des empreintes en lumière rasante.

Les résultats sont regroupés au tableau ci-après.

| Variation | Surface | Nombre | L totale | Longueur Moyenne |
|---|---|---|---|---|
| T2H / J1 | -22,1 | -18,5 | -24,2 | -7,7 |
| J28 / J1 | -13,4 | -10,8 | -14,5 | -13,8 |
| J56 / J1 | -29,5 | -19,3 | -26,0 | -18,9 |

Les résultats sont exprimés en pourcentage de variation par rapport à la valeur initiale.

L'analyse effectuée à T2H (2 heures) montre une diminution statistiquement significative de la surface de la peau occupée par les rides, du nombre de rides et de leur longueur, ce qui démontre un effet lissant sur la peau après 2 heures d'application.

L'analyse à J28 (28 jours) montre une diminution statistiquement significative de la longueur totale des rides.

L'analyse à J56 (56 jours) montre une diminution statistiquement significative de la surface occupée, du nombre de rides, de la longueur moyenne et de la longueur totale, démontrant un effet potentiel antirides après 56 jours d'application quotidienne de la composition de l'invention.

Ces résultats confirment l'effet antirides amélioré de la composition de l'invention combinant l'extrait de graines de mimosa, l'extrait de souci et le palmitoyl pentapeptide-3.

## Revendications

1. Utilisation d'un extrait de graines de mimosa pour la préparation d'une composition cosmétique et/ou dermatologique pour application topique, destinée à lutter contre les signes du vieillissement cutané, **caractérisée en ce que** l'extrait de graines de mimosa est un extrait d'Acacia dealbata, d'Acacia farnesiana ou d'Acacia decurrens.

2. Utilisation selon la revendication 1, **caractérisée en ce que** l'extrait de graines de mimosa est obtenu par macération dans l'eau de graines de mimosa pulvérisées.

3. Utilisation selon l'une quelconque des revendications précédentes, **caractérisée en ce que** l'extrait de mimosa présente les caractéristiques suivantes :
- matières sèches 2 à 4%
- densité à 22°C 0,980 à 1,020
- indice de réfraction à 22°C 1,330 à 1,350
- pH 5,7 à 7,2
- teneur en protéines totale >0,1% (par rapport aux matières sèches)

4. Utilisation selon l'une quelconque des revendications précédentes, **caractérisée en ce que** la composition comprend entre 0,001 et 15% en poids d'extrait de mimosa.

5. Utilisation selon l'une quelconque des revendications précédentes, **caractérisée en ce que** la composition comprend en outre un extrait de souci.

6. Utilisation selon la revendication 5, **caractérisée en ce que** l'extrait de souci est un extrait de Calendula officinalis ou un extrait de Calendula arvensis.

7. Utilisation selon la revendication 5, **caractérisée en ce que** l'extrait de souci est obtenu à partir de pétales de souci.

8. Utilisation selon la revendication 5, **caractérisée en ce que** l'extrait de souci est obtenu par macération hydro-glycolique de fleurs pulvérisées.

9. Utilisation selon la revendication 8, **caractérisée en ce que** l'extrait de souci est obtenu par macération de fleurs pulvérisées dans un mélange butylène glycol/eau.

10. Utilisation selon la revendication 5, **caractérisée en ce que** l'extrait de souci présente les caractéristiques suivantes :
- eau / butylène glycol 50/50
- matières sèches 1,5 à 3,0%
- flavonoïdes 0,2 à 3,0% (sur matière sèche, exprimé en rutine)

11. Utilisation selon la revendication 5, **caractérisée en ce que** la composition comprend entre 0,001 et 0,5% en poids d'extrait de souci.

12. Utilisation selon l'une quelconque des revendications précédentes, **caractérisée en ce que** la composition comprend en outre des quantités appropriées d'une ou plusieurs substances choisies parmi la Vitamine A palmitate, la Vitamine C ou ses dérivés, les protéines de graines d'amarante (Amarantus caudatus), les globulines de pois ou des mucilages tels que le mucilage de fruits de baobab, l'huile de Calophylum, l'huile d'Echium, un palmitoyl pentapeptide-3 ou des dérivés tels que le palmitoyl GHK et le palmitoyl GQPR ou le palmitoyl VGVAPG associé à un céramide-2, ainsi qu'un céramide, une association comprenant du palmitoyl pentapeptide-3 et un extrait de Siegesbeckia orientalis, les polyphénols, tels que les polyphénols de cacao, l'acide oléanolique, une antihyaluronidase telle que Echinacine B, l'Imperata cylindrica, un extrait de graines de lotus, de graines de coquelicot, de racine de guimauve, les extraits de fenouil.

13. Procédé cosmétique pour combattre les signes du vieillissement cutané, consistant à appliquer sur les zones de la peau affectées une composition contenant entre 0,001 et 15 % en poids d'extrait de graines de mimosa par rapport au poids total de la composition, l'extrait de graines de mimosa étant un extrait d'Acacia dealbata, d'Acacia farnesiana ou d'Acacia decurrens.

14. Procédé selon la revendication 13, **caractérisé en ce que** la composition comprend en outre entre 0,001 et 0,5 % en poids d'extrait de souci par rapport au poids total de la composition.

15. Composition cosmétique utile contre les signes du vieillissement cutané, **caractérisée en ce qu'**elle comprend en combinaison un extrait de graines de mimosa choisi parmi un extrait d'Acacia dealbata, d'Acacia farnesiana et d'Acacia decurrens, et un extrait de souci.

16. Composition selon la revendication 15, **caractérisée en ce qu'**elle comprend en outre un palmitoyl pentapeptide-3.

## Patentansprüche

1. Verwendung eines Mimosensamenextrakts für die Zubereitung einer kosmetischen und/oder dermatologischen Zusammensetzung zur topischen Anwendung, die zur Bekämpfung der Zeichen der Hautalterung bestimmt ist, **dadurch gekennzeichnet, dass** der Mimosensamenextrakt ein Extakt aus Acacia dealbata, Acacia farnesiana oder Acacia decurrens ist.

2. Verwendung nach Anspruch 1, **dadurch gekennzeichnet, dass** der Mimosensamenextrakt durch Mazeration von pulverisierten Mimosensamen in Wasser hergestellt wird.

3. Verwendung nach einem der vorangehenden Ansprüche, **dadurch gekennzeichnet, dass** der Mimosenextrakt die folgenden Eigenschaften aufweist:
- Trockenmassen 2 bis 4 %
- Dichte bei 22 °C 0,980 bis 1,020
- Refraktionsindex bei 22 °C 1,330 bis 1,350
- pH 5,7 bis 7,2
- Proteingehalt insgesamt >0,1 % (im Verhältnis zu den Trockenmassen).

4. Verwendung nach einem der vorangehenden Ansprüche, **dadurch gekennzeichnet, dass** die Zusammensetzung zwischen 0,001 und 15 % Gew.-% Mimosenextrakt umfasst.

5. Verwendung nach einem der vorangehenden Ansprüche, **dadurch gekennzeichnet, dass** die Zusammensetzung ferner einen Ringelblumenextrakt umfasst.

6. Verwendung nach Anspruch 5, **dadurch gekennzeichnet, dass** der Ringelblumenextrakt ein Extrakt aus Calendula officinalis oder aus Calendula arvensis ist.

7. Verwendung nach Anspruch 5, **dadurch gekennzeichnet, dass** der Ringelblumenextrakt aus Ringelblumenblütenblättern gewonnen wird.

8. Verwendung nach Anspruch 5, **dadurch gekennzeichnet, dass** der Ringelblumenextrakt durch hydroglykolische Mazeration pulverisierter Blüten gewonnen wird.

9. Verwendung nach Anspruch 8, **dadurch gekennzeichnet, dass** der Ringelblumenextrakt durch Mazeration pulverisierter Blüten in einem Butylenglycol-Wasser-Gemisch gewonnen wird.

10. Verwendung nach Anspruch 5, **dadurch gekennzeichnet, dass** der Ringelblumenextrakt die folgenden Eigenschaften aufweist:
- Wasser/Butylenglycol 50/50
- Trockenmassen 1,5 bis 3,0 %
- Flavonoide 0,2 bis 3,0 % (der Trockenmasse, ausgedrückt in Rutin).

11. Verwendung nach Anspruch 5, **dadurch gekennzeichnet, dass** die Zusammensetzung zwischen 0,001 und 0,5 Gew.-% Ringelblumenextrakt umfasst.

12. Verwendung nach einem der vorangehenden Ansprüche, **dadurch gekennzeichnet, dass** die Zusammensetzung ferner geeignete Mengen einer oder mehrerer Substanzen umfasst, die aus dem Vitamin-A-Palmitat, dem Vitamin C oder seinen Derivaten, den Proteinen der Amaranthsamen (Amarantus caudatus), den Erbsenglobulinen oder den Schleimen wie dem Schleim von Baobabfrüchten, dem Calophylumöl, dem Echiumöl, einem Palmitoylpentapeptid-3 oder Derivaten wie dem Palmitoyl GHK und dem Palmitoyl GQPR oder dem Palmitoyl VGVAPG kombiniert mit einem Ceramid-2 sowie einem Ceramid, einer Kombination, die Palmitoylpentapeptid-3 und einen Extrakt aus Siegesbeckia orientalis, den Polyphenolen wie den Kakaopolyphenolen, der Oleanolsäure, einer Antihyaluronidase wie Echinacin B, der Imperata cylindrica, einem Extrakt von Lotussamen, von Mohnsamen, von Eibischwurzel, den Fenchelextrakten ausgewählt sind.

13. Kosmetisches Verfahren zur Bekämpfung der Zeichen der Hautalterung, das darin besteht, auf die betroffenen Hautbereiche eine Zusammensetzung aufzutragen, die zwischen 0,001 und 15 Gew.-% Mimosensamenextrakt im Verhältnis zum Gesamtgewicht der Zusammensetzung enthält, wobei der Mimosensamenextrakt ein Extrakt aus Acacia dealbata, Acacia farnesiana oder Acacia decurrens ist.

14. Verfahren nach Anspruch 13, **dadurch gekennzeichnet dass** die Zusammensetzung ferner zwischen 0,001 und 0,5 Gew.-% Ringelblumenextrakt im Verhältnis zum Gesamtgewicht der Zusammensetzung umfasst.

15. Kosmetische Zusammensetzung gegen die Zeichen der Hautalterung, **dadurch gekennzeichnet, dass** sie in Kombination einen Mimosensamenextrakt, ausgewählt aus einem Extrakt aus Acacia dealbata, Acacia farnesiana oder Acacia decurrens, und einen Ringelblumenextrakt umfasst.

16. Zusammensetzung nach Anspruch 15, **dadurch gekennzeichnet, dass** sie ferner ein Palmitoylpentapeptid-3 umfasst.

## Claims

1. The use of an extract of mimosa seeds to prepare a cosmetic and/or dermatological composition for topical application, intended to combat signs of skin ageing, **characterized in that** the extract of mimosa seeds is an extract of *Acacia dealbata, Acacia farnesiana* or *Acacia decurrens.*

2. The use according to claim 1, **characterized in that** the extract of mimosa seeds is obtained by macerating powdered mimosa seeds in water.

3. The use according to any of the preceding claims, **characterized in that** the extract of mimosa has the following characteristics:
- dry matter 2 to 4 %
- density at 22°C 0.980 to 1.020
- refractive index at 22°C 1.330 to 1.350
- pH 5.7 to 7.2
- total protein content >0.1 %
(relative to dry matter).

4. The use according to any of the preceding claims, **characterized in that** the composition comprises between 0.001 and 15 % by weight of mimosa extract.

5. The use according to any of the preceding claims, **characterized in that** the composition further comprises a marigold extract.

6. The use according to claim 5, **characterized in that** the marigold extract is an extract of *Calendula officinalis* or an extract of *Calendula arvensis.*

7. The use according to claim 5, **characterized in that** the marigold extract is obtained from marigold petals.

8. The use according to claim 5, **characterized in that** the marigold extract is obtained by hydro-glycolic maceration of powdered flowers.

9. The use according to claim 8, **characterized in that** the marigold extract is obtained by maceration of powered flowers in a butylene glycol/water mixture.

10. The use according to claim 5, **characterized in that** the marigold extract has the following characteristics:
- water/ Butylene glycol 50:50
- dry matter 1.5 to 3.0 %
- flavonoids 0.2 to 3.0 %
(on dry matter expressed in rutin).

11. The use according to claim 5, **characterized in that** the composition comprises between 0.001 and 0.5 % by weight of marigold extract.

12. The use according to any of the preceding claims, **characterized in that** the composition further comprises suitable quantities of one or more substances selected from among Vitamin A palmitate, Vitamin C or the derivatives thereof, proteins of amaranth seeds *(Amarantus caudatus),* pea globulin or mucilage globulin such as baobab fruit mucilage, Calophylum oil, Echium oil, palmitoyl pentapeptide-3 or derivatives such as palmitoyl-GHK and palmitoyl-GQPR or palmitoyl-VGVAPG associated with a ceramide-2, and a ceramide, an association comprising palmitoyl pentapeptide-3 and an extract of *Siegesbeckia orientalis,* polyphenols such as polyphenols of cocoa, oleanolic acid, an antihyaluronidase such as Echinacin B, *Imperata cylindrica,* an extract of lotus seeds, poppy seeds, Marshmallow root, fennel extracts.

13. A cosmetic method for combating signs of skin ageing consisting of applying to regions of affected skin a composition containing between 0.001 and 15 % by weight of extract of mimosa seeds relative to the total weight of the composition, the extract of mimosa seeds being an extract of *Acacia dealbata, Acacia farnesiana* or *Acacia decurrens.*

14. The method according to claim 13, **characterized in that** the composition further comprises between 0.001 and 0.5 % by weight of marigold extract relative to the total weight of the composition.

15. A cosmetic composition useful against signs of skin ageing **characterized in that** it comprises in combination an extract of mimosa seeds selected from an extract of *Acacia dealbata, Acacia farnesiana* and *Acacia decurrens,* and a marigold extract.

16. The composition according to claim 15, **characterized in that** it further comprises a palmitoyl pentapeptide-3.
